# EUROPEAN PATENT SPECIFICATION

(11) **EP 2 563 325 B1**
(45) Date of publication and mention of the grant of the patent: **06.04.2016**
(21) Application number: 11714036.8
(22) Date of filing: 12.04.2011
(51) Int. Cl.: A61K 8/91, A61Q 5/00, C08F 220/28, C08F 220/40, A61K 8/81, A61K 8/86

(54) **Method of protecting hair and polymer for use therein**
Verfahren zum Schutz von Haar und ein Polymer zur Verwendung darin
Procédé de protection des cheveux et une polymère pour l'utilisation dans celui-ci

(30) Priority: 28.04.2010 EP 10161308
(43) Date of publication of application: 06.03.2013
(73) Proprietor: Unilever PLC, London EC4Y 0DN (GB); Unilever N.V., 3013 AL Rotterdam (NL)
(72) Inventor: HADDLETON, David, Mark, Kenilworth Warwickshire CV8 2TP (GB); KHOSHDEL, Ezat, Wirral Merseyside CH63 3JW (GB); SLAVIN, Stacy, Hamilton South Lanarkshire Scotland ML3 9NT (GB)
(74) Representative: James, Helen Sarah
(86) International application number: PCT/EP2011/055729
(87) International publication number: WO 2011/134785

(56) References cited:
- EP-A2- 0 863 161
- WO-A1-01/47047
- WO-A1-2006/026229
- WO-A1-2008/058989
- WO-A2-01/44388
- WO-A2-2008/013757
- WO-A2-2009/126960
- GB-A- 1 318 170
- JP-A- 6 065 328
- JP-A- 58 172 618
- JP-A- 62 209 512
- US-A1- 2005 123 700
- US-A1- 2007 212 641
- LUKASZCZYK JAN ET AL: "Preparation of functional microspheres by polymerization using macromonomers", POLIMERY, INSTYTUT CHEMII PRZEMYSOWEJ, WARSAW, PL, vol. 45, no. 10, 1 January 2000 (2000-01-01), pages 674-679, XP009139057, ISSN: 0032-2725

## Description

The present invention relates to new polymers and their use for protecting the hair fibre and other keratineous materials from damaging treatment.

Damage to the hair typically manifests itself in cuticle and protein loss from the hair fibre, hair fibre dryness, hair fibre brittleness, hair breakage and frayed or split ends.

Many every day activities damage the hair. For example, environmental sources of hair damage include exposure to UV and chlorine. Chemical sources of hair damage include treatments such as bleaching, perming and straightening, and overly frequent washing with harsh surfactant-based cleansing shampoo compositions. Mechanical sources of hair damage include excessive brushing and combing and prolonged use of heated appliances for drying and styling the hair.

WO2008/058989 describes a permanent way of preventing damage to the hair by covalently bonding to the hair a polymeric compound comprising polyethylene glycol.

The present invention addresses the problem of hair damage prevention, but has the additional advantage that it describes ways of repairing damaged hair. The polymers used in this process are also relatively easy to produce.

### Summary of the Invention

The present invention relates to a hair care composition comprising a polymeric compound as described in claim 4.

The invention also provides the use of the polymeric compound described above for derivatising hair.

Also claimed are polymers consisting of poly[poly(ethylene glycol) methyl ether methacrylate-co-allyl methacrylate.

### Detailed description

The present invention relates to a method of protecting hair by covalently bonding to the hair a polymeric compound as described in claim 4. Modification of proteins with polyethyleneglycol is commonly known as PEGylation.

One way of describing a preferred polymeric compound comprising polyethylene glycol, is of a central backbone with a cluster of PEG polymers or PEG segments.

It is preferred if the molecular weight Mw of the polymer is from 1,000 to 100,000 more preferably from 5,000 to 60,000, most preferably from 10,000 to 50,000.

It is also preferable if the polymeric compound comprises a central backbone in which the number of methacrylate groups is from 1 to 20, preferably from 2 to 10.

The polymer type is poly[poly(ethylene glycol) methyl ether methacrylate-co-allyl methacrylate]alternatively known as ω-vinyl-poly[Poly(ethylene glycol) methyl ether methacrylate-*co*-allyl methacrylate].

Such polymers are usually prepared by catalytic chain transfer polymerisation (CCTP) and are thus known as CCTP polymers.

The level of polymeric compound comprising polyethylene glycol is preferably from 0.01 to 15 wt.% of the total composition, more preferably from 0.1 to 10 wt.%.

### Product Form

The final product form of hair treatment compositions according to the invention may suitably be, for example, shampoos, conditioners, sprays, mousses, gels, waxes or lotions.

The product may be a leave on product (left on the hair for at least 30 minutes) or a wash off product, in particular a shampoo or post-wash conditioner.

The pH of the formulations of the invention are in the range from pH 3 to pH 11, more preferably used at a pH from 3 to 8.

### Hair Treatment Composition Base Formulation

The composition of the invention may be a shampoo formulation. Shampoo compositions preferably comprise one or more cleansing surfactants, which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

The total amount of anionic cleansing surfactant in shampoo compositions of the invention is generally from 5 to 30, preferably from 6 to 20, more preferably from 8 to 16 wt% of the total composition.

### Co-surfactant

The shampoo composition can optionally include co-surfactants, preferably an amphoteric or zwitterionic surfactant, which can be included in an amount ranging from 0 to about 8, preferably from 1 to 4 wt%.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred co-surfactant is a nonionic surfactant, which can be included in an amount ranging from 0 to 8 wt%, preferably from 2 to 5 wt% of the total composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)n

wherein R is a branched or straight chain C₅ to C₂₀ alkyl or alkenyl group, G is a saccharide group and n is from 1 to 10.

The shampoo composition can also optionally include one or more cationic co-surfactants included in an amount ranging from 0.01 to 10, more preferably from 0.05 to 5, most preferably from 0.05 to 2 wt% of the total composition. Useful cationic surfactants are described herein in relation to conditioner compositions.

The total amount of surfactant (including any co-surfactant, and/or any emulsifier) in shampoo compositions of the invention is generally from 5 to 50, preferably from 5 to 30, more preferably from 10 to 25 wt%.

### Cationic Deposition Polymer

A cationic polymer is a preferred ingredient, especially in shampoo compositions of the invention.

The cationic polymer may be a homopolymer or be formed from two or more types of monomers. The molecular weight of the polymer will generally be between 5 000 and 10 000 000 Dalton, typically at least 10 000 and preferably from 100 000 to 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

The cationic deposition polymer will generally be present in compositions of the invention at levels of from 0.01 to 5, preferably from 0.02 to 1, more preferably from 0.04 to 0.5 percent by weight of the composition.

### Conditioning Surfactant

Conditioner compositions usually comprise one or more conditioning surfactants which are cosmetically acceptable and suitable for topical application to the hair.

Suitable conditioning surfactants are selected from cationic surfactants, used singly or in a mixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺ (X)

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

### Fatty Materials

Conditioner compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15 wt%, preferably from 0.1 to 10 wt% of the total composition. The weight ratio of cationic surfactant to fatty alcohol is suitably from 10:1 to 1:10, preferably from 4:1 to 1:8, optimally from 1:1 to 1:7.

### Suspending Agents

In a preferred embodiment, the shampoo compositions of this invention further comprises from 0.1 to 5 wt% of a suspending agent for the coated particles. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

The suspending agent is preferably a polymeric suspending agent.

### Styling polymers

If the composition is a styling product it is preferred if a styling polymer is present

The hair styling polymer if present is preferably present in the compositions of the invention in an amount of from 0.001% to 10% by weight, more preferably from 0.1% to 10% by weight, such as from 1% to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

The amount of the polymer may range from 0.5 to 10%, preferably 0.75 to 6% by weight based on total weight of the composition.

### Adjuvants

The compositions of the present invention may also contain adjuvants suitable for hair care. Generally such ingredients are included individually at a level of up to 2, preferably up to 1 wt% of the total composition.

Suitable hair care adjuvants, include amino acids and ceramides.

The invention will now be illustrated by the following non-limiting Examples.

Example of the invention are illustrated by a number, comparative Examples are illustrated by a letter.

Polymers of the invention were prepared by CCTP as described in figure 1.

### Examples:

### Preparation of CCTP co-polymer PEG-AMA (M_{w} 3800)

Methanol (38mL) and demineralised water (38mL) were placed into a round bottomed flask with a magnetic stirrer bar, sealed with a suba-seal, para-film and purged with nitrogen for 2hrs prior to the experiment starting. Di(ethylene glycol) methyl ether methacrylate (DEGMEMA) monomer (29.41 mL, 0.16 Mol, 188.22 g.mol⁻¹) was placed into a round bottom flask, along with allyl methacrylate (AMA) monomer (1.07mL, 0.008 Mol, 126 g.mol⁻¹) sealed with a suba-seal, para-film and purged with nitrogen for 2hrs. Initiator 4,4'-azobis(4-cyano-valeric acid) (CVA) (0.28g, 8.21x10⁻⁴ Mol, 280.28 g.mol⁻¹) was placed into a round bottom flask with a magnetic stirrer bar, sealed with a suba-seal, para-film and purged with nitrogen for 2hrs. Bis(boron difluorodimethylglyoximate) cobalt (II) (CoBF, chain transfer agent) (3.06mg, 7.97x10⁻⁶ Mol, 503 g. mol⁻¹) was placed into a round bottom flask with a magnetic stirrer bar, sealed with a suba-seal, para-film and purged with nitrogen for 2hrs.

The MeOH and H₂O mixture was cannulated into the round bottom flask containing CVA and the solution was heated to 80°C in an oil bath with a fuzzy-logic temperature controller. The DEGMEMA/AMA monomer mixture was cannulated into the round bottom flask containing CoBF and 3.2mL of this solution was transferred into the solvent/initiator round bottom flask using a degassed syringe. A feed pump was used to supply the remaining monomer/CoBF mixture into the solvent/CVA mixture at a rate of 0.43mL/min (feed completed after 60 minutes) and samples for analysis were taken periodically using a degassed syringe. After five hours the reaction was removed from the oil bath and air was bubbled through the reaction solution.

### Purification

The remaining monomer and solvent in the reaction were removed using a vacuum pump with the collection flask cooled in liquid nitrogen. The sample flask was heated to 30°C to stop the solution from freezing.

### Preparation of PEGylated polymers

Comparative PEGylated polymers were prepared as in WO2008/058989.

### DSC Studies

### Sample preparation

European hair fibres were bleached two times with L'Oreal platifiz precision powder and Oxydant crème (1:1.5) for 30min, rinsed completely with running water from the tap, and naturally dried overnight before the next application. These hair fibres were immersed in 1% active solution and water (as a control) at pH 5.5 for 1 h respectively, rinsed with distilled water for 30 sec and naturally dried overnight. And then they were cut into approximatel 2mm length with scissors.

### DSC method

About 6 mg of sample was weighted into a pressure resistant (25 bar), stainless steel, large volume pan (60 µl capacity). 50 µl of water was added and the pan was sealed. Samples were then mixed using a rotary mixer and left overnight to allow the water to equilibrate throughout the sample. Samples were run through a temperature programme of 120-180°C at a rate of 5°C/ min in 30ml/min nitrogen atmosphere. The helix transition temperature was collected and analyzed with one-way ANOVA. Each sample was carried out at least four times.

### DSC Data

The DSC data of bleached hair fibre as well as bleached and PEGylated hair fibres is given in Table 1 below. A high denaturation temperature (Td) indicates that the hair is not denatured.

**Table 1 - Comparison of the Denaturation data of with CCTP Co-Polymer PEG-AMA & PEGylated hair**

| **Sample** | **Average Denaturation temperature (°C)** | **Average Enhancement (°C)** |
|---|---|---|
| **Virgin hair fibre** | **153.39** | **-** |
| **Damaged hair fibre (Bleached)** | **137.19** | **Baseline** |
| **CCTP Co-Polymer PEG-AMA MW 3800 treated hair SS-1, m SS-2, SS-3 Example 1** | **147.20** | **10.01** |
| **12K-PEGylated hair JN-1 Example A** | **142.67** | **5.48** |
| **45K-PEGylated hair JN-2 Example B** | **142.52** | **5.33** |

The Example of the invention shows a 10°C enhancement compared to the damaged hair.

Comparative Examples (Examples A and B) in which the hair is merely pegylated preventing hair damage to a lesser extent.

## Claims

1. A method of protecting hair by applying to hair a composition comprising a polymeric compound consisting of poly[poly(ethylene glycol) methyl ether methacrylate-co-allyl methacrylatel.

2. A method according to any preceding claim in which the molecular weight Mw of the polymer is from 5,000 to 60,000.

3. A method according to any preceding claim in which the polymeric compound comprises a central backbone in which the number of methacrylate groups is from 2 to 10.

4. A polymeric compound consisting of poly[poly(ethylene glycol) methyl ether methacrylate-co-allyl methacrylate].

## Patentansprüche

1. Verfahren zum Schutz von Haar, bei dem auf das Haar eine Zusammensetzung aufgetragen wird, die eine polymere Verbindung umfasst, die aus Poly[poly(ethylenglycol)methylethermethacrylat-co-allylmethacrylat] besteht.

2. Verfahren nach einem vorhergehenden Anspruch, worin das Molekulargewicht Mw des Polymers 5.000 bis 60.000 beträgt.

3. Verfahren nach irgendeinem vorhergehenden Anspruch, wobei die polymere Verbindung eine zentrale Kette umfasst, in der die Zahl der Methacrylat-Gruppen 2 bis 10 beträgt.

4. Polymere Verbindung, die aus Poly[poly(ethylenglycol)methylethermethacrylat-co-allylmethacrylat] besteht.

## Revendications

1. Procédé de protection des cheveux par application aux cheveux d'une composition comprenant un composé polymère constitué de poly[méthacrylate de poly(éthylène glycol) méthyléther-co-méthacrylate d'allyle].

2. Procédé selon l'une quelconque des revendications précédentes, dans lequel la masse moléculaire Mw du polymère est de 5 000 à 60 000.

3. Procédé selon l'une quelconque des revendications précédentes, dans lequel le composé polymère comprend un squelette central dans lequel le nombre de groupes méthacrylate est de 2 à 10.

4. Composé polymère constitué de poly[méthacrylate de poly(éthylène glycol) méthyléther-co-méthacrylate d'allyle].
